(19) Europäisches Patentamt
European Patent Office
Office européen des brevets

(11) EP 1 332 123 B1

(12) **FASCICULE DE BREVET EUROPEEN**

(45) Date de publication et mention de la délivrance du brevet:
**14.12.2005 Bulletin 2005/50**

(21) Numéro de dépôt: **01982569.4**

(22) Date de dépôt: **26.10.2001**

(51) Int Cl.$^7$: **C07C 45/48**, C07C 49/167, C07C 49/80

(86) Numéro de dépôt international:
**PCT/FR2001/003337**

(87) Numéro de publication internationale:
**WO 2002/036536 (10.05.2002 Gazette 2002/19)**

(54) **PROCEDE DE PREPARATION DE CETONES FLUOREES**

VERFAHREN ZUR HERSTELLUNG VON FLUORIERTEN KETONEN

METHOD FOR PREPARING FLUORINATED KETONES

(84) Etats contractants désignés:
**AT BE CH CY DE DK ES FI FR GB GR IE IT LI LU MC NL PT SE TR**

(30) Priorité: **06.11.2000 FR 0014176**

(43) Date de publication de la demande:
**06.08.2003 Bulletin 2003/32**

(73) Titulaire: **RHODIA CHIMIE**
**92512 Boulogne-Billancourt (FR)**

(72) Inventeur: **JACQUOT, Roland**
**F-69340 FRANCHEVILLE (FR)**

(74) Mandataire: **Esson, Jean-Pierre et al**
**Rhodia Services,**
**Direction de la Propriété Industrielle,**
**Centre de Recherches de Lyon**
**BP 62**
**85, avenue des Frères Perret**
**69192 Saint-Fons Cédex (FR)**

(56) Documents cités:

**EP-A- 0 085 996**
**EP-A- 0 283 660**
**EP-A- 0 352 674**
**DE-A- 2 758 113**
**FR-A- 2 368 457**
**US-A- 2 697 729**
**US-A- 4 172 851**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 1 332 123 B1

Printed by Jouve, 75001 PARIS (FR)

## Description

La présente invention a pour objet un procédé de préparation de cétones fluorées. L'invention s'applique plus particulièrement aux cétones présentant au moins un atome de fluor, de préférence trois atomes de fluor en position α par rapport au groupe carbonyle.

Il est connu de préparer une cétone α-trifluorée selon un procédé qui consiste à faire réagir un composé organométallique avec l'acide trifluoroacétique ou ses esters [Chem. L. S. et al, J. Fluorine Chem. VIII, p. 117 (1981)].

Ce procédé souffre de plusieurs inconvénients. Il comporte plusieurs étapes, préparation du composé organométallique à partir du bromobenzène puis réaction avec de l'acide trifluoroacétique à basse température (- 78°C) et hydrolyse ce qui complique sa mise en oeuvre et il est difficilement transposable à l'échelle industrielle. De plus, le rendement réactionnel n'est pas satisfaisant en raison de la formation de sous-produits.

On a décrit dans US 4 172 851, un procédé de préparation d'une cétone partiellement fluorée qui consiste à faire réagir un anhydride d'acide non fluoré et un anhydride d'acide carboxylique perfluoré, en l'absence de catalyseur.

On a également décrit dans FR 2 368 457, un procédé de préparation : d'une cétone aliphatique perfluorée par réaction d'un sel d'acide perfluorocarboxylique avec un fluorure d'acide perfluorocarboxylique, dans un solvant polaire aprotique, à une température de 20°C à 200°C.

Il est également connu d'une manière générale, de préparer une cétone à partir d'un ou plusieurs acides carboxyliques selon la réaction de Piria qui consiste à faire réagir le ou les acides carboxyliques en phase gazeuse, en présence d'un oxyde métallique qui peut être choisi parmi les oxydes de métaux alcalins, alcalino-terreux, les oxydes des métaux des groupes IIIb, IVb et Vb.

Il s'avère que la préparation d'une cétone fluorée et plus particulièrement d'une cétone α-trifluorée selon la réaction de Piria n'est pas décrite dans la littérature car l'on assiste à haute température à une défluoration du réactif de départ conduisant ainsi à une fluoration du catalyseur.

Il a maintenant été trouvé et c'est ce qui constitue l'objet de la présente invention, un procédé de préparation d'une cétone fluorée répondant à la formule générale :

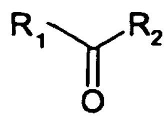

(I)

dans laquelle :

- $R_1$ et $R_2$, identiques ou différents, représentent un groupe hydrocarboné comportant de 1 à 40 atomes de carbone qui peut être un groupe aliphatique acyclique saturé ou insaturé, linéaire ou ramifié ; un groupe carbocyclique ou hétérocyclique saturé, insaturé ou aromatique, monocyclique ou polycyclique; un enchaînement des groupes précités.
- au moins l'un des groupes $R_1$ et $R_2$, ne comprend pas d'atomes d'hydrogène sur l'atome de carbone en position α par rapport au groupe carbonyle,
- au moins l'un des groupes $R_1$ et $R_2$ comprend un ou plusieurs atomes de fluor,

ledit procédé étant caractérisé par le fait que l'on fait réagir, en phase gazeuse, à une température comprise entre 200°C et 500°C

- un acide carboxylique de formule (II) :

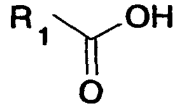

(II)

dans laquelle $R_1$ est telle que définie ci-dessus,

- et un acide carboxylique de formule (III) :

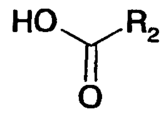

**(III)**

dans laquelle $R_2$ est telle que définie ci-dessus,
en présence d'un catalyseur comprenant un mélange d'oxyde de thorium et d'oxyde de terre rare ou de l'alumine.

Par "terre rare", on entend les lanthanides ayant un numéro atomique de 57 à 71 et l'yttrium ainsi que le scandium.

Conformément à l'invention, on part d'acides carboxylique de formules (II) et (III) mais l'invention inclut la mise en oeuvre de dérivés d'acides carboxyliques tels que anhydrides d'acides carboxyliques ou cétènes correspondants.

L'invention permet d'obtenir des cétones symétriques si dans la formule (I), R, est identique à $R_2$ et des cétones disymétriques si $R_1$ est différent de $R_2$.

Plus précisément, dans les formules (I) à (III), $R_1$ et $R_2$ représentent un groupe hydrocarboné ayant de 1 à 20 atomes de carbone qui peut être un groupe aliphatique acyclique saturé ou insaturé, linéaire ou ramifié ; un groupe carbocyclique ou hétérocyclique saturé, insaturé ou aromatique, monocyclique ou polycyclique ; un groupe aliphatique saturé ou insaturé, linéaire ou ramifié, porteur d'un substituant cyclique.

$R_1$ et $R_2$ représentent préférentiellement un groupe aliphatique acyclique saturé linéaire ou ramifié ayant de préférence de 1 à 12 atomes de carbone, et encore plus préférentiellement de 1 à 4 atomes de carbone.

L'invention n'exclut pas la présence d'une insaturation sur la chaîne hydrocarbonée telle qu'une ou plusieurs doubles liaisons qui peuvent être conjuguées ou non, ou une triple liaison.

La chaîne hydrocarbonée peut être éventuellement interrompue par un hétéroatome (par exemple, oxygène ou soufre) ou par un groupe fonctionnel dans la mesure où celui-ci ne réagit pas et l'on peut citer en particulier un groupe tel que notamment -CO-.

La chaîne hydrocarbonée peut être éventuellement porteuse d'un ou plusieurs substituants (par exemple, halogène, ester) dans la mesure où ils n'interfèrent pas avec la réaction de cétonisation.

Le groupe aliphatique acyclique, saturé ou insaturé, linéaire ou ramifié peut être éventuellement porteur d'un substituant cyclique. Par cycle, on entend un cycle carbocyclique ou hétérocyclique, saturé, insaturé ou aromatique.

Le groupe aliphatique acyclique peut être relié au cycle par un lien valentiel, un hétéroatome ou un groupe fonctionnel tels que oxy, carbonyle, carboxy ou sulfonyle.

Comme exemples de substituants cycliques, on peut envisager des substituants cycloaliphatiques, aromatiques ou hétérocycliques, notamment cycloaliphatiques comprenant 6 atomes de carbone dans le cycle ou benzéniques, ces substituants cycliques étant eux-mêmes éventuellement porteurs d'un substituant quelconque dans la mesure où ils ne gênent pas les réactions intervenant dans le procédé de l'invention. On peut mentionner en particulier, les groupes alkyle, alkoxy ayant de 1 à 4 atomes de carbone.

Parmi les groupes aliphatiques porteurs d'un substituant cyclique, on vise plus particulièrement les groupes cycloalkylalkyle, par exemple, cyclohexylalkyle ou les groupes aralkyle ayant de 7 à 12 atomes de carbone, notamment benzyle ou phényléthyle.

Dans les formules (I) à (III), $R_1$ et $R_2$ peuvent également représenter un groupe carbocyclique, saturé ou non ayant de préférence 5 ou 6 atomes de carbone dans le cycle; un groupe hétérocyclique, saturé ou non, comportant notamment 5 ou 6 atomes dans le cycle dont 1 ou 2 hétéroatomes tels que les atomes d'azote, de soufre et d'oxygène ; un groupe carbocyclique ou hétérocyclique aromatique, monocyclique, de préférence, phényle, pyridyle, pyrazolyle, imidazolyle ou polycyclique condensé ou non, de préférence, naphtyle.

Dès lors que l'un des groupes $R_1$ et $R_2$ comprend un cycle, celui-ci peut être également substitué. La nature du substituant peut être quelconque dans la mesure où il n'interfère pas avec la réaction principale. Le nombre de substituants est généralement au plus de 4 par cycle mais le plus souvent égal à 1 ou 2.

Parmi toutes les significations données précédemment pour $R_1$ et $R_2$, ils représentent de préférence un groupe alkyle linéaire ou ramifié ayant de 1 à 12 atomes de carbone, de préférence de 1 à 6 atomes de carbone ou un groupe phényle.

Comme mentionné précédemment, au moins l'un des groupes $R_1$ et $R_2$, ne comprend pas d'atomes d'hydrogène sur l'atome de carbone en position $\alpha$ par rapport au groupe carbonyle.

Ainsi, l'un des atomes de carbone en position $\alpha$ par rapport au groupe carbonyle est un atome de carbone

tertiaire. Il peut être représenté par la formule ($R_3$) ($R_4$) ($R_5$) C - dans laquelle $R_3$, $R_4$ et $R_5$ représentent notamment, un atome d'halogène, de préférence, un atome de fluor ; un groupe alkyle linéaire ou ramifié ayant de 1 à 6 atomes de carbone ; les groupes $R_3$, $R_4$ et $R_5$, pouvant également former un cycle, par exemple, un groupe phényle.

Comme exemples d'acides carboxyliques comprenant un atome de carbone tertiaire, on peut mentionner les acides carboxyliques perfluorés.

En effet, l'invention s'applique tout particulièrement à la préparation de cétones fluorées à partir d'acides carboxyliques dont l'un deux est au moins un acide carboxylique aliphatique fluoré répondant plus particulièrement à la formule (IIa) :

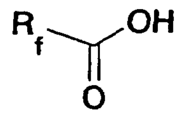

(IIa)

dans laquelle :

- $R_f$ représente une chaîne perfluorée de formule :

$$-[CF_2]_p-CF_3$$

dans ladite formule, p représente un nombre allant de 0 à 10.

Les acides carboxyliques aliphatiques préférés répondent à la formule (IIa) dans laquelle Rf représente préférentiellement les groupes :

$$-CF_3$$

$$-CF_2-CF_3$$

L'invention s'applique également à la préparation de cétones fluorées à partir d'acides carboxyliques aromatiques fluorés répondant plus particulièrement à la formule (IIb) :

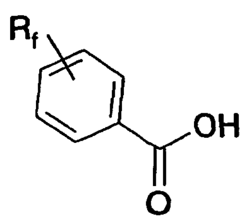

(IIb)

dans laquelle $R_f$ a la signification donnée précédemment mais représente de préférence, un groupe trifluorométhyle.

Comme exemples d'acides carboxyliques de formule (IIa) ou (IIb), on peut citer :

- l'acide trifluoroacétique,
- l'acide pentafluoroacétique,
- les acides o- m- et p-trifluorométhylbenzoïque.

Pour ce qui est de l'acide carboxylique de formule (III), on met en oeuvre préférentiellement ceux répondant à la formule (III) dans laquelle $R_2$ représente :

- un groupe alkyle linéaire ou ramifié, ayant de 1 à 20 atomes carbone, de préférence de 1 à 12 atomes de carbone,
- un groupe cycloalkyle ayant de 3 à 8 atomes de carbone, de préférence un groupe cyclopentyle ou cyclohexyle,
- un groupe aryle ayant de 6 à 12 atomes de carbone, de préférence un groupe phényle ou naphtyle,
- un groupe arylalkyle ayant de 7 à 12 atomes de carbone, de préférence un groupe benzyle.

Comme exemples d'acides carboxyliques de formule (IIb), on peut mentionner plus particulièrement :

- l'acide acétique,
- l'anhydride acétique,
- l'acide propanoïque,
- l'acide butanoïque,
- l'acide pentanoïque.

Conformément au procédé de l'invention, on effectue la réaction de cétonisation de l'acide carboxylique, en présence d'un catalyseur comprenant un mélange d'oxyde de thorium et d'oxyde de terre rare ou de l'alumine.
Il s'agit plus particulièrement d'un oxyde d'une terre rare choisie parmi les lanthanides, l'yttrium le scandium et leurs mélanges, de préférence les lanthanides tels que le lanthane, le cérium, le praséodyme, le néodyme, le samarium, l'europium, le gadolinium, le terbium, le dysprosium, l'holmium, l'erbium, le thulium, l'ytterbium et le lutécium.
On choisit parculièrement les mélanges d'oxyde de thorium et d'oxyde de cérium.
On met en oeuvre avantageusement des mélanges comprenant de 20 à 40 % d'oxyde de thorium et de 60 à 80 % d'oxyde de terre rare, de préférence de cérium.
Un autre type d'élément catalytique convenant à la mise en oeuvre du procédé de l'invention est l'aluminium
On peut utiliser toutes les alumines du commerce mais l'on préfère mettre en oeuvre l'alumine gamma.
On met en oeuvre avantageusement une alumine ayant une surface spécifique d'au moins 100 $m^2/g$, de préférence comprise entre 150 et 350 $m^2/g$, et plus préférentiellement comprise entre 200 et 280 $m^2/g$.
Son volume poreux varie le plus souvent entre 0,2 et 1,0 $cm^3/g$, de préférence entre 0,4 et 0,9 $cm^3/g$.
Le volume poreux comme la surface spécifique sont mesurés selon la méthode BRUNEAU-EMMETT-TELLER décrite dans le périodique «The Journal of American Society 60,309 (1938) ».
Généralement, l'alumine est mise en oeuvre sous la forme d'extrudés ayant un diamètre de 0,5 à 5 mm, de préférence, de 1 à 3 mm et une longueur de 2 à 100 mm, de préférence, de 5 à 10 mm. Il est également possible qu'elle soit sous forme de billes, pastilles ou granulés.
La matière active est apportée soit directement sous la forme d'oxyde ou bien obtenu après calcination d'un support imprégné à l'aide d'une solution apportant les éléments précités sous forme d'hydroxyde, de sel simple ou double, minéral ou organique.
Il est possible de faire appel à un hydroxyde, à un sel minéral de préférence, nitrate, sulfate, oxysulfate, halogénure, oxyhalogénure, silicate, carbonate, oxalate ou à un sel organique de préférence, acétylacétonate ; alcoolate et encore plus préférentiellement méthylate ou éthylate ; carboxylate en $C_1$-$C_6$ tels que acétate ou bien encore les carboxylates dérivés des acides gras en $C_8$-$C_{22}$ et notamment les stéarate, palmitate, myristate et laurate.
La matière active est avantageusement mise en oeuvre sous une forme oxyde.
La nature du support peut être variable. C'est ainsi que peuvent convenir à la présente invention, notamment, les charbons actifs, les gels de silice, les mélanges silice-alumine, l'alumine, les argiles (et plus particulièrement, le kaolin, le talc ou la montmorillonite), la bauxite, la magnésie et la terre de diatomées.
Dans le catalyseur, la teneur en phase active représente de 5 à 100 % du poids du catalyseur.
Les catalyseurs peuvent se présenter sous différentes formes dans le procédé de l'invention : poudre, produits mis en forme tels que granulés (par exemple, extrudés ou billes), pastilles, qui sont obtenus par extrusion, moulage, compactage ou tout autre type de procédé connu.
Pour préparer le catalyseur supporté utile à la mise en oeuvre du procédé de la présente invention, on peut recourir à des techniques classiques, connues en elles-mêmes, de préparation de catalyseurs métalliques supportés. On peut se référer, notamment, pour la préparation des différents catalyseurs à l'ouvrage : [J. F. LEPAGE "Catalyse de contact", conception, préparation et mise en oeuvre des catalyseurs industriels, Edition Technip (1978)].
Une méthode connue pour la préparation d'oxydes métalliques fixés sur support inerte consiste à dissoudre dans l'eau un sel du métal choisi, et à verser la solution obtenue sur des particules du support inerte activé. L'ensemble est alors calciné entre 400°C et 600°C de façon à assurer la transformation du sel métallique en oxyde métallique fixé sur support inerte.
Conformément au procédé de l'invention, la réaction de cétonisation est conduite en phase gazeuse, en mettant en contact l'acide carboxylique (II) avec l'acide carboxylique (III), en présence du catalyseur tel que défini.
Généralement, la quantité des acides carboxyliques mis en oeuvre est telle que le rapport entre le nombre de moles d'acide carboxylique (III) et le nombre de moles d'acide carboxylique (II) varie entre 1 et 20, de préférence

entre 3 et 8.

Conformément à l'invention, le procédé est conduit en phase gazeuse. On entend par cette expression que les différents réactifs sont vaporisés dans les conditions réactionnelles mais le procédé n'exclut pas la présence d'une éventuelle phase liquide résultant soit des propriétés physiques des réactifs, soit d'une mise en oeuvre sous pression ou de l'utilisation d'un solvant organique.

Le gaz vecteur est optionnel et est en général un gaz ou un mélange de gaz non réactifs dans les conditions de la réaction. On peut faire appel à des gaz tel que l'azote, l'air, l'argon ou l'hélium. Avantageusement, le rapport volumique entre le gaz vecteur et l'acide carboxylique (II) varie entre 0 et 10, de préférence entre 0,1 et 2,0.

La température de la réaction de cétonisation est comprise de préférence de 250°C et 450°C, et encore plus préférentiellement entre 280°C et 300°C.

La pression réactionnelle est de préférence la pression atmosphérique mais il est également possible de conduire le procédé sous pression réduite pouvant descendre jusqu'à 100 mm de mercure lorsque les réactifs de départ sont peu volatils.

Conformément au procédé de l'invention, on vaporise les réactifs de départ à savoir les acides carboxyliques. Ils sont introduits au contact du catalyseur, de préférence entraînés par un gaz vecteur.

La WHSV (weight hourly space velocity) est comprise entre 0,05 et 1,5 $h^{-1}$, de préférence entre 0,1 et 0,8 $h^{-1}$.

En pratique, la réaction est réalisée aisément en continu par passage du flux gazeux dans un réacteur tubulaire contenant le catalyseur.

On commence par préparer le lit catalytique qui est constitué par la phase active catalytique éventuellement déposée sur un support (par exemple, verre fritté) ce qui permet la circulation des gaz sans élution du catalyseur. Ensuite, les réactifs sont mis en oeuvre et plusieurs variantes sont possibles.

Il est possible de vaporiser chacun des réactifs (II) et (III), dans des chambres différentes, puis d'effectuer le mélange dans une chambre de mélange et d'introduire le flux gazeux résultant sur le catalyseur. Le gaz vecteur peut être introduit en parallèle audit flux gazeux ou bien au niveau de la chambre de mélange.

Une autre variante consiste à préparer une solution comprenant les réactifs (II) et (III), puis à vaporiser ledit mélange et à l'introduire sur le catalyseur, en parallèle avec le gaz vecteur.

Un autre mode de réalisation pratique de l'invention consiste à faire fondre l'un des acides carboxyliques en le chauffant jusqu'à sa température de fusion et l'on fait passer au-dessus un flux gazeux comprenant l'autre acide carboxylique. Ce flux se sature du premier acide carboxylique et il est mis alors en contact avec le catalyseur.

Un autre mode d'exécution de l'invention est de faire appel à un solvant organique, inerte dans les conditions réactionnelles et qui est choisi de telle sorte qu'il solubilise l'acide carboxylique (II) et l'acide carboxylique (III) mis en oeuvre.

On fait appel de préférence selon l'invention à un solvant aprotique ayant un point d'ébullition supérieur à 60°C et, de préférence, compris entre 60°C et 300°C.

A titre de solvants aprotiques susceptibles d'être mis en oeuvre dans le procédé de l'invention, on peut citer les hydrocarbures aliphatiques ou aromatiques comme l'hexane, l'heptane, le cyclohexane, le benzène, le toluène, les xylènes.

On peut également utiliser plusieurs solvants.

La quantité d'acide carboxylique (II) mise en oeuvre dans le solvant est généralement telle que le rapport molaire solvant/acide carboxylique (II) soit compris entre 0 et 20 et, de préférence, entre 0 et 5.

Ainsi, on prépare une solution organique comprenant l'acide carboxylique (II), l'acide carboxylique (III), puis l'on vaporise ledit mélange et on l'introduit sur le catalyseur, en parallèle avec le gaz vecteur.

En fin de réaction, on condense l'ensemble des gaz et l'on sépare les réactifs non réagis et les produits obtenus, par distillation ou cristallisation fractionnée. Il est également possible de séparer ceux-ci, par condensation fractionnée. Dans le cas plus particulier de la trifluoroacétone, cette dernière est récupérée sous forme d'hydrate, en piégeant le flux gazeux dans l'eau.

Le procédé de l'invention permet d'obtenir préférentiellement une cétone répondant à la formule (I) de type perfluorée.

Le procédé de l'invention est parfaitement bien adapté à la préparation de la trifluoroacétone.

Le procédé peut être mis en oeuvre en continu.

Le mode préféré de réalisation de l'invention est de mettre en oeuvre un catalyseur de type alumine. Le catalyseur de l'invention peut être aisément regénéré par traitement à l'air entre 450°C et 600°C, de préférence entre 480°C et 500°C. Il est maintenu sous air jusqu'à ce qu'il n'y ait plus de dioxyde de carbone dégagé.

La régénération du catalyseur peut se faire séparément ou in situ.

Le catalyseur régénéré conserve toutes ses performances catalytiques.

Les exemples qui suivent illustrent l'invention sans toutefois la limiter.

EXEMPLES

Dans les différents exemples qui suivent, les abréviations TT, RR ont la signification suivante :

$$\text{Taux de transformation} = TT = \frac{\text{nombre de moles de l'acide carboxylique* transformées}}{\text{nombre de moles d'acide carboxylique* introduites}} \text{ en \%}$$

$$\text{Rendement Réel} = RR = \frac{\text{nombre de moles de cétone formées}}{\text{nombre de moles d'acide carboxylique* introduites}} \text{ en \%}$$

* : l'acide concerné est l'acide mis en oeuvre en défaut.

Exemple 1

Dans un réacteur en verre de 25 mm de diamètre, on introduit successivement :

- 5 ml de quartz de granulométrie 300-600 µm
- 3 ml de catalyseur $CeO_2$ /$ThO_2$ (62/38)
- 5 ml de quartz

On place le réacteur dans un four électrique et on raccorde un tube collecteur au bas du réacteur.
On fait passer un courant de 3 l/h d'azote et l'on chauffe à 350°C pendant une heure.
Une fois l'activation terminée, on ramène la température à 280°C et on injecte par un pousse seringue 3,2 ml/h d'un mélange acide trifluoroacétique, à 10 % p/p dans l'acide acétique.
Les gaz effluents sont piégés dans un piège contenant de l'eau pour former l'hydrate de la trifluoroacétone.
Après 3 heures de réaction, on obtient par chromatographie en phase gazeuse, les résultats suivant :

- TT acide trifluoroacétique : 83 %
- RR trifluoroacétone : 42 %

Exemple 2

On reproduit l'exemple 1 à la différence près que l'on met en oeuvre 1 ml de catalyseur $CeO_2$/ $ThO_2$.
On obtient les résultats suivants:

- TT acide trifluoroacétique : 23%
- RR trifluoroacétone : 4 %

Exemple 3

On reproduit l'exemple 1 avec 3 ml de catalyseur $CeO_2$ /$ThO_2$ (72/28)
On obtient les résultats suivants :

- TT acide trifluoroacétique : 78 %
- RR trifluoroacétone : 35 %

Exemple 4

Dans un réacteur en verre de 30 ml , on introduit 20 ml d'alumine gamma de surface spécifique = 280 $m^2$/g et le volume poreux total est de 0,8 $cm^3$/g sous forme d'extrudés (1,2 mm de diamètre, 5 à 10 mm de long).
On chauffe le réacteur à 280°C avec un débit d'azote de 2 litres par heure et l'on maintient 1 h dans ces conditions.
On injecte un mélange d'acide trifluoroacétique dans l'anhydride acétique à 10% P/P à un débit de 11 ml/h.
Les gaz réactionnels sont ensuite partiellement condensés dans un ballon maintenu à 25°C pour récupérer les réactifs n'ayant pas réagi, puis dans un piège contenant de l'eau pour récupérer la trifluoroacétone formée sous forme d'hydrate.
Après 5 heures de réaction, l'analyse par RMN nous donne les résultats suivants :

- TT acide trifluoroacétique : 83%
- RR trifluoroacétone : 70%

Exempte 5

On répète l'exemple précédent mais la réaction est conduite à 230 °C
Dans ces conditions, les résultats obtenus sont les suivants :

- TT acide trifluoroacétique : 47 %
- RR trifluoroacétone : 31 %

Exemple 6

On répète l'exemple 4 précédent mais l'on injecte 6 ml/h d'un mélange à 20% P/P d'acide trifluoroacétique dans l'anhydride acétique.
Dans ces conditions, les résultats obtenus sont les suivants :

- TT acide trifluoroacétique : 79 %
- RR trifluoroacétone : 64 %

Exemple 7

On reproduit l'exemple 4 précédent mais l'on utilise une alumine alpha.
Dans ces conditions, les résultats obtenus sont les suivants :

- TT acide trifluoroacétique : 24 %
- RR trifluoroacétone : 5 %

Exemple 8

Recyclage du catalyseur.

Apres désactivation du catalyseur de l'exemple 4, celui-ci est calciné sous un courant d'air de 3 l/h pendant 15 heures à 500°C.
On ramène ensuite la température à 280°C et l'on recommence les injections dans les mêmes conditions.
On obtient les résultats suivants :

- TT acide trifluoroacétique : 81 %
- RR trifluoroacétone : 71 %

Le catalyseur est recyclable sans perte notable d'activite.

## Revendications

1. Procédé de préparation d'une cétone fluorée répondant à la formule générale :

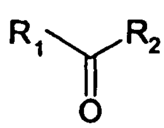

(I)

dans laquelle :

- $R_1$ et $R_2$, identiques ou différents, représentent un groupe hydrocarboné comportant de 1 à 40 atomes de

carbone qui peut être un groupe aliphatique acyclique saturé ou insaturé, linéaire ou ramifié ; un groupe carbocyclique ou hétérocyclique saturé, insaturé ou aromatique, monocyclique ou polycyclique; un enchaînement des groupes précités.

- au moins l'un des groupes $R_1$ et $R_2$, ne comprend pas d'atomes d'hydrogène sur l'atome de carbone en position $\alpha$ par rapport au groupe carbonyle,
- au moins l'un des groupes $R_1$ et $R_2$ comprend un ou plusieurs atomes de fluor,

ledit procédé étant **caractérisé par le fait que** l'on fait réagir, en phase gazeuse, à une température comprise entre 200°C et 500°C,

- un acide carboxylique de formule (II) :

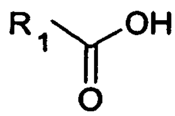

(II)

dans laquelle $R_1$ est telle que définie ci-dessus,

- et un acide carboxylique de formule (III) :

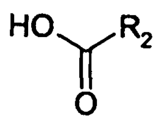

(III)

dans laquelle $R_2$ est telle que définie ci-dessus,
en présence d'un catalyseur comprenant un mélange d'oxyde de thorium et d'oxyde de terre rare ou de l'alumine.

**2.** Procédé selon la revendication 1 **caractérisé par le fait que** l'élément métallique est déposé sur un support, de préférence choisi parmi la silice, l'alumine, les mélanges silice-alumine, les argiles, la bauxite, la magnésie et la terre de diatomées.

**3.** Procédé selon la revendication 1 **caractérisé par le fait que** le catalyseur est un mélange d'oxyde de thorium et d'oxyde de cérium.

**4.** Procédé selon l'une des revendications 1 et 2 **caractérisé par le fait que** le catalyseur est un mélange comprenant de 20 à 40 % d'oxyde de thorium et de 60 à 80 % d'oxyde de terre rare, de préférence de cérium.

**5.** Procédé selon la revendication 1 **caractérisé par le fait que** le catalyseur est l'alumine gamma ayant de préférence une surface spécifique comprise entre 150 et 350 $m^2$/g, et préférentiellement entre 200 et 280 $m^2$/g.

**6.** Procédé selon la revendication 1 **caractérisé par le fait que** les acides carboxyliques répondent aux formules (II) ou (III) dans lesquelles $R_1$ et $R_2$ représentent :

- un groupe aliphatique acyclique linéaire ou ramifié,
- un groupe aliphatique acyclique porteur d'un substituant cyclique, éventuellement substitué, de préférence, un cycle benzénique, pouvant être relié au cycle par un lien valentiel, un hétéroatome ou un groupe fonctionnel,
- un groupe carbocyclique, saturé ou non ayant de préférence 5 ou 6 atomes de carbone dans le cycle; un groupe hétérocyclique, saturé ou non, comportant notamment 5 ou 6 atomes dans le cycle dont 1 ou 2 hétéroatomes tels que les atomes d'azote, de soufre et d'oxygène ; un groupe carbocyclique ou hétérocyclique aromatique, monocyclique, de préférence, phényle ou pyridyle ou polycyclique condensé ou non, de préférence, naphtyle.

**7.** Procédé selon la revendication 6 **caractérisé par le fait que** les acides carboxyliques répondent aux formules (II) ou (III) dans lesquelles $R_1$ et $R_2$ représentent un groupe alkyle linéaire ou ramifié ayant de 1 à 12 atomes de carbone, de préférence de 1 à 6 atomes de carbone ou un groupe phényle.

**8.** Procédé selon l'une des revendications 1 , 6 et 7 **caractérisé par le fait que** l'acide de carboxylique de formule (II) comprend un atome de carbone tertiaire situé en position a par rapport au groupe carbonyle et qui peut être représenté par la formule ($R_3$) ($R_4$) ($R_5$) C - dans laquelle $R_3$, $R_4$ et $R_5$ représentent un atome d'halogène, de préférence, un atome de fluor ; un groupe alkyle linéaire ou ramifié ayant de 1 à 6 atomes de carbone ; les groupes $R_3$, $R_4$ et $R_5$, pouvant également former un cycle, de préférence, un groupe phényle.

**9.** Procédé selon la revendication 8 **caractérisé par le fait que** $R_3$, $R_4$ et $R_5$ représentent un atome de fluor ou un groupe phényle.

**10.** Procédé selon la revendication 1 **caractérisé par le fait que** l'acide carboxylique de formule (II) est un acide carboxylique aliphatique fluoré répondant à la formule (IIa) :

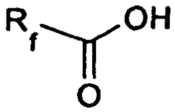

**(IIa)**

dans laquelle :

- $R_f$ représente une chaîne perfluorée de formule :

$$- [CF_2]_p -CF_3$$

dans ladite formule, p représente un nombre allant de 0 à 10.

**11.** Procédé selon la revendication 10 **caractérisé par le fait que** l'acide carboxylique répond à la formule (IIa) dans laquelle Rf représente préférentiellement les groupes :

$$- CF_3$$

$$- CF_2-CF_3$$

**12.** Procédé selon la revendication 1 **caractérisé par le fait que** l'acide carboxylique de formule (II) est un acide carboxylique aromatique fluoré répondant à la formule (IIb) :

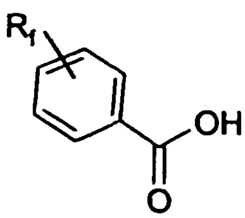

**(IIb)**

dans laquelle $R_f$ a la signification donnée précédemment dans la revendication 8, de préférence, un groupe trifluorométhyle.

**13.** Procédé selon la revendication 1 **caractérisé par le fait que** l'acide carboxylique de formule (IIa) ou (IIb) est choisi parmi :

- l'acide trifluoroacétique,
- l'acide pentafluoroacétique,
- les acides o- m- et p-trifluorométhylbenzoïque.

**14.** Procédé selon la revendication 1 **caractérisé par le fait que** l'acide carboxylique de formule (III) est un acide carboxylique répondant à la formule (III) dans laquelle $R_2$ représente :

- un groupe alkyle linéaire ou ramifié, ayant de 1 à 20 atomes carbone, de préférence de 1 à 12 atomes de carbone,
- un groupe cycloalkyle ayant de 3 à 8 atomes de carbone, de préférence un groupe cyclopentyle ou cyclohexyle,
- un groupe aryle ayant de 6 à 12 atomes de carbone, de préférence un groupe phényle ou naphtyle,
- un groupe arylalkyle ayant de 7 à 12 atomes de carbone, de préférence un groupe benzyle.

**15.** Procédé selon la revendication 1 **caractérisé par le fait que** l'acide carboxylique de formule (III) est choisi parmi :

- l'acide acétique,
- l'anhydride acétique,
- l'acide propanoïque,
- l'acide butanoïque,
- l'acide pentanoïque.

**16.** Procédé selon la revendication 1 **caractérisé par le fait que** l'acide carboxylique de formule (II) est l'acide trifluoroacétique et l'acide de formule (III) est l'acide acétique.

**17.** Procédé selon la revendication 1 **caractérisé par le fait que** la quantité des acides carboxyliques mis en oeuvre est telle que le rapport entre le nombre de moles d'acide carboxylique (III) et le nombre de moles d'acide carboxylique (II) varie entre 1 et 20, de préférence entre 3 et 8.

**18.** Procédé selon la revendication 1 **caractérisé par le fait que** la WHSV (weight hourly space velocity) est comprise entre 0,05 et 1,5 $h^{-1}$, de préférence entre 0,1 et 0,8 $h^{-1}$.

**19.** Procédé selon la revendication 1 **caractérisé par le fait que** l'on met en oeuvre un gaz vecteur ou un mélange de gaz non réactifs dans les conditions de la réaction, de préférence l'azote, l'air, l'argon ou l'hélium.

**20.** Procédé selon la revendication 19 **caractérisé par le fait que** le rapport volumique entre le gaz vecteur et l'acide carboxylique (II) varie entre 0 et 10, de préférence entre 0,1 et 2,0.

**21.** Procédé selon la revendication 1 **caractérisé par le fait que** l'on met en oeuvre un solvant organique, de préférence aprotique, qui solubilise les acides carboxyliques.

**22.** Procédé selon la revendication 1 **caractérisé par le fait que** la température de la réaction de cétonisation est comprise entre 250°C et 450°C et de préférence entre 280°C et 300°C.

**23.** Procédé selon la revendication 1 **caractérisé par le fait que** la pression réactionnelle est la pression atmosphérique.

**24.** Procédé selon la revendication 1 **caractérisé par le fait que** l'on vaporise les réactifs de départ à savoir les acides carboxyliques puis ils sont introduits au contact du catalyseur, de préférence entraînés par un gaz vecteur.

## Patentansprüche

**1.** Verfahren zur Herstellung eines fluorierten Ketons, das der allgemeinen Formel:

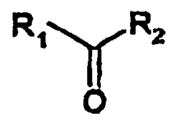

·(I)

entspricht, worin:

- $R_1$ und $R_2$, die gleich oder verschieden sind, eine Kohlenwasserstoffgruppe mit 1 bis 40 Kohlenstoffatomen darstellen, die eine lineare oder verzweigte, gesättigte oder ungesättigte, acyclische aliphatische Gruppe; eine monocyclische oder polycyclische, gesättigte, ungesättigte oder aromatische, carbocyclische oder heterocyclische Gruppe; eine Verknüpfung der zuvor angeführten Gruppen sein kann,
- wenigstens eine der Gruppen $R_1$ und $R_2$ keine Wasserstoffatome an dem Kohlenstoffatom in $\alpha$-Position, bezogen auf die Carbonylgruppe, umfasst,
- wenigstens eine der Gruppen $R_1$ und $R_2$ ein oder mehrere Fluoratome umfasst,

wobei besagtes Verfahren **dadurch gekennzeichnet ist, dass** man in der Gasphase bei einer Temperatur zwischen 200 °C und 500 °C

- eine Carbonsäure der Formel (II)

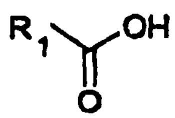

(II)

worin $R_1$ wie oben definiert ist,
- und eine Carbonsäure der Formel (III):

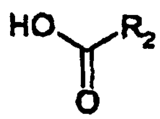

(III)

worin $R_2$ wie oben definiert ist,
in Gegenwart eines Katalysators, der ein Gemisch von Thoriumoxid und Seltenerdoxid oder Aluminiumoxid umfasst, umsetzt.

2. Verfahren gemäß Anspruch 1, **dadurch gekennzeichnet, dass** das metallische Element auf einem Träger abgeschieden ist, der vorzugsweise ausgewählt ist unter Siliciumdioxid, Aluminiumoxid, den Siliciumdioxid-Aluminiumoxid-Gemischen, den Tonen, Bauxit, Magnesiumoxid und Diatomeenerde.

3. Verfahren gemäß Anspruch 1, **dadurch gekennzeichnet, dass** der Katalysator ein Gemisch von Thoriumoxid und Ceroxid ist.

4. Verfahren gemäß einem der Ansprüche 1 und 2, **dadurch gekennzeichnet, dass** der Katalysator ein Gemisch ist, das 20 bis 40 % Thoriumoxid und 60 bis 80 % Seltenerdoxid, vorzugsweise Ceroxid umfasst.

5. Verfahren gemäß Anspruch 1, **dadurch gekennzeichnet, dass** der Katalysator gamma-Aluminiumoxid, vorzugsweise mit einer spezifischen Oberfläche zwischen 150 und 350 $m^2/g$ und vorzugsweise zwischen 200 und 280

$m^2/g$ ist.

**6.** Verfahren gemäß Anspruch 1, **dadurch gekennzeichnet, dass** die Carbonsäuren den Formeln (II) oder (III) entsprechen, worin $R_1$ und $R_2$ darstellen:

- eine lineare oder verzweigte acyclische aliphatische Gruppe,
- eine acyclische aliphatische Gruppe, die einen gegebenenfalls substituierten cyclischen Substituenten trägt, vorzugsweise ein Benzenring, die mit dem Ring über eine Valenzbindung, ein Heteroatom oder eine funktionelle Gruppe verbunden sein kann,
- eine gesättigte oder nicht gesättigte carbocyclische Gruppe mit vorzugsweise 5 oder 6 Kohlenstoffatomen im Ring; eine gesättigte oder nicht gesättigte heterocyclische Gruppe mit insbesondere 5 oder 6 Atomen im Ring, davon 1 oder 2 Heteroatome, wie das Stickstoff-, Schwefel- und Sauerstoffatom; eine aromatische carbocyclische oder heterocyclische Gruppe, die eine monocyclische Gruppe, vorzugsweise Phenyl oder Pyridyl, oder eine kondensierte oder nicht kondensierte polycyclische Gruppe, vorzugsweise Naphthyl ist.

**7.** Verfahren gemäß Anspruch 6, **dadurch gekennzeichnet, dass** die Carbonsäuren den Formeln (II) oder (III) entsprechen, worin $R_1$ und $R_2$ eine lineare oder verzweigte Alkylgruppe mit 1 bis 12 Kohlenstoffatomen, vorzugsweise 1 bis 6 Kohlenstoffatomen, oder eine Phenylgruppe darstellen.

**8.** Verfahren gemäß einem der Ansprüche 1, 6 und 7, **dadurch gekennzeichnet, dass** die Carbonsäure der Formel (II) ein tertiäres Kohlenstoffatom umfasst, das sich in $\alpha$-Position, bezogen auf die Carbonylgruppe, befindet und das durch die Formel $(R_3)(R_4)(R_5)C$ dargestellt werden kann, worin $R_3$, $R_4$ und $R_5$ ein Halogenatom, vorzugsweise ein Fluoratom; eine lineare oder verzweigte Alkylgruppe mit 1 bis 6 Kohlenstoffatomen darstellen; wobei die Gruppen $R_3$, $R_4$ und $R_5$ auch einen Ring, vorzugsweise eine Phenylgruppe bilden können.

**9.** Verfahren gemäß Anspruch 8, **dadurch gekennzeichnet, dass** $R_3$, $R_4$ und $R_5$ ein Fluoratom oder eine Phenylgruppe darstellen.

**10.** Verfahren gemäß Anspruch 1, **dadurch gekennzeichnet, dass** die Carbonsäure der Formel (II) eine fluorierte aliphatische Carbonsäure ist, die der Formel (IIa):

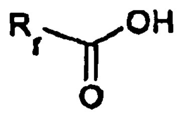

**(IIa)**

entspricht, worin:

- $R_f$ eine perfluorierte Kette der Formel:

$$-[CF_2]_p-CF_3$$

darstellt, wobei in besagter Formel p eine Zahl von 0 bis 10 darstellt.

**11.** Verfahren gemäß Anspruch 10, **dadurch gekennzeichnet, dass** die Carbonsäure der Formel (IIa) entspricht, worin Rf vorzugsweise die Gruppen:

$$-CF_3$$

$$-CF_2-CF_3$$

darstellt.

**12.** Verfahren gemäß Anspruch 1, **dadurch gekennzeichnet, dass** die Carbonsäure der Formel (II) eine fluorierte aromatische Carbonsäure ist, die der Formel (IIb)

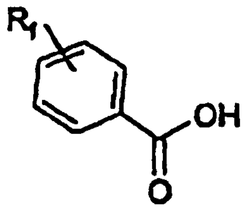

**(IIb)**

entspricht, worin $R_f$ die zuvor in Anspruch 8 angegebene Bedeutung hat, vorzugsweise eine Trifluormethylgruppe.

**13.** Verfahren gemäß Anspruch 1, **dadurch gekennzeichnet, dass** die Carbonsäure der Formel (IIa) oder (IIb) ausgewählt ist unter:

- Trifluoressigsäure,
- Pentafluoressigsäure,
- den o-, m- und p-Trifluormethylbenzoesäuren.

**14.** Verfahren gemäß Anspruch 1, **dadurch gekennzeichnet, dass** die Carbonsäure der Formel (III) eine Carbonsäure ist, die der Formel (III) entspricht, worin $R_2$ darstellt:

- eine lineare oder verzweigte Alkylgruppe mit 1 bis 20 Kohlenstoffatomen, vorzugsweise 1 bis 12 Kohlenstoffatomen,
- eine Cycloalkylgruppe mit 3 bis 8 Kohlenstoffatomen, vorzugsweise eine Cyclopentyl- oder Cyclohexylgruppe,
- eine Arylgruppe mit 6 bis 12 Kohlenstoffatomen, vorzugsweise eine Phenyl- oder Naphthylgruppe,
- eine Arylalkylgruppe mit 7 bis 12 Kohlenstoffatomen, vorzugsweise eine Benzylgruppe.

**15.** Verfahren gemäß Anspruch 1, **dadurch gekennzeichnet, dass** die Carbonsäure der Formel (III) ausgewählt ist unter:

- Essigsäure,
- Essigsäureanhydrid,
- Propansäure,
- Butansäure,
- Pentansäure.

**16.** Verfahren gemäß Anspruch 1, **dadurch gekennzeichnet, dass** die Carbonsäure der Formel (II) Trifluoressigsäure ist und die Säure der Formel (III) Essigsäure ist.

**17.** Verfahren gemäß Anspruch 1, **dadurch gekennzeichnet, dass** die Menge der eingesetzten Carbonsäuren so ist, dass das Verhältnis zwischen der Anzahl an Molen Carbonsäure (III) und der Anzahl an Molen Carbonsäure (II) zwischen 1 und 20, vorzugsweise zwischen 3 und 8 variiert.

**18.** Verfahren gemäß Anspruch 1, **dadurch gekennzeichnet, dass** die WHSV (weight hourly space velocity) zwischen 0,05 und 1,5 $h^{-1}$, vorzugsweise zwischen 0,1 und 0,8 $h^{-1}$ liegt.

**19.** Verfahren gemäß Anspruch 1, **dadurch gekennzeichnet, dass** man ein Trägergas oder ein Gemisch von Gasen, die unter den Reaktionsbedingungen nicht reaktiv sind, vorzugsweise Stickstoff, Luft, Argon oder Helium, einsetzt.

**20.** Verfahren gemäß Anspruch 19, **dadurch gekennzeichnet, dass** das Volumenverhältnis zwischen dem Trägergas und der Carbonsäure (II) zwischen 0 und 10, vorzugsweise zwischen 0,1 und 2,0 variiert.

**21.** Verfahren gemäß Anspruch 1, **dadurch gekennzeichnet, dass** man ein organisches, vorzugsweise aprotisches

Lösungsmittel einsetzt, das die Carbonsäuren löst.

**22.** Verfahren gemäß Anspruch 1, **dadurch gekennzeichnet, dass** die Temperatur der Ketonisierungsreaktion zwischen 250 °C und 450 °C und vorzugsweise zwischen 280 °C und 300 °C liegt.

**23.** Verfahren gemäß Anspruch 1, **dadurch gekennzeichnet, dass** der Reaktionsdruck der Atmosphärendruck ist.

**24.** Verfahren gemäß Anspruch 1, **dadurch gekennzeichnet, dass** man die Ausgangsreagenzien, nämlich die Carbonsäuren, verdampft, dann werden sie, vorzugsweise durch ein Trägergas mitgeschleppt, in Kontakt des Katalysators gebracht.

**Claims**

**1.** Process for the preparation of a fluoroketone corresponding to the general formula:

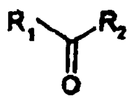

(I)

in which:

- $R_1$ and $R_2$, which are identical or different, represent a hydrocarbon group comprising from 1 to 40 carbon atoms which can be a saturated or unsaturated and linear or branched acyclic aliphatic group; a saturated, unsaturated or aromatic, monocyclic or polycyclic, carbocyclic or heterocyclic group; or a sequence of the abovementioned groups,
- at least one of the $R_1$ and $R_2$ groups does not comprise hydrogen atoms on the carbon atom in the $\alpha$ position with respect to the carbonyl group,
- at least one of the $R_1$ and $R_2$ groups comprises one or more fluorine atoms,

the said process being **characterized in that**:

- a carboxylic acid of formula (II):

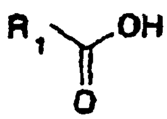

(II)

  in which $R_1$ is as defined above,
- and a carboxylic acid of formula (III):

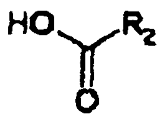

(III)

in which $R_2$ is as defined above,

are reacted in the gas phase at a temperature of between 200°C and 500°C in the presence of a catalyst comprising a mixture of thorium oxide and of rare earth metal oxide or of alumina.

**2.** Process according to Claim 1, **characterized in that** the metal element is deposited on a support preferably chosen from silica, alumina, silica/alumina mixtures, clays, bauxite, magnesia and diatomaceous earth.

**3.** Process according to Claim 1, **characterized in that** the catalyst is a mixture of thorium oxide and of cerium oxide.

**4.** Process according to either of Claims 1 and 2, **characterized in that** the catalyst is a mixture comprising from 20 to 40% of thorium oxide and from 60 to 80% of rare earth metal oxide, preferably cerium oxide.

**5.** Process according to Claim 1, **characterized in that** the catalyst is γ-alumina preferably having a specific surface of between 150 and 350 $m^2/g$ and preferably between 200 and 280 $m^2/g$.

**6.** Process according to Claim 1, **characterized in that** the carboxylic acids correspond to the formulae (II) and (III) in which $R_1$ and $R_2$ represent:

- a linear or branched acyclic aliphatic group,
- an acyclic aliphatic group carrying an optionally substituted cyclic substituent, preferably a benzene ring, which can be connected to the ring via a valency bond, a heteroatom or a functional group,
- a saturated or unsaturated carbocyclic group preferably having 5 or 6 carbon atoms in the ring; a saturated or unsaturated heterocyclic group comprising in particular 5 or 6 atoms in the ring, including 1 or 2 heteroatoms, such as nitrogen, sulphur and oxygen atoms; or an aromatic carbocyclic or heterocyclic group which is monocyclic, preferably a phenyl or pyridyl group, or which is condensed or noncondensed polycyclic, preferably a naphthyl group.

**7.** Process according to Claim 6, **characterized in that** the carboxylic acids correspond to the formulae (II) and (III) in which $R_1$ and " $R_2$ represent a linear or branched alkyl group having from 1 to 12 carbon atoms, preferably from 1 to 6 carbon atoms, or a phenyl group.

**8.** Process according to one of Claims 1, 6 and 7, **characterized in that** the carboxylic acid of formula (II) comprises a tertiary carbon atom situated in the α position with respect to the carbonyl group which can be represented by the formula $(R_3)$ $(R_4)$ $(R_5)$c- in which $R_3$, $R_4$ and $R_5$ represent a halogen atom, preferably a fluorine atom, or a linear or branched alkyl group having from 1 to 6 carbon atoms, it also being possible for the $R_3$, $R_4$ and $R_5$ groups to form a ring, preferably a phenyl group.

**9.** Process according to Claim 8, **characterized in that** $R_3$, $R_4$ and $R_5$ represent a fluorine atom or a phenyl group.

**10.** Process according to Claim 1, **characterized in that** the carboxylic acid of formula (II) is a fluorinated aliphatic carboxylic acid corresponding to the formula (IIa):

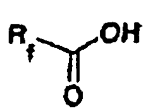

**(IIa)**

in which:

- $R_f$ represents a perfluorinated chain of formula:

$$-[CF_2]_p-CF_3$$

in which p represents a number ranging from 0 to 10.

**11.** Process according to Claim 10, **characterized in that** the carboxylic acid corresponds to the formula (IIa) in which $R_f$ preferably represents the following groups:

$$-CF_3$$

$$-CF_2-CF_3.$$

**12.** Process according to Claim 1, **characterized in that** the carboxylic acid of formula (II) is a fluorinated aromatic carboxylic acid corresponding to the formula (IIb):

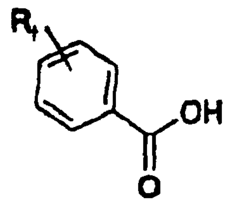

**(IIb)**

in which $R_f$ has the meaning given above in Claim 10, preferably a trifluoromethyl group.

**13.** Process according to Claim 1, **characterized in that** the carboxylic acid of formula (IIa) or (IIb) is chosen from:

- trifluoroacetic acid,
- pentafluoroacetic acid,
- o-, m- and p-trifluoromethylbenzoic acids.

**14.** Process according to Claim 1, **characterized in that** the carboxylic acid of formula (III) is a carboxylic acid corresponding to the formula (III) in which $R_2$ represents:

- a linear or branched alkyl group having from 1 to 20 carbon atoms, preferably from 1 to 12 carbon atoms,
- a cycloalkyl group having from 3 to 8 carbon atoms, preferably a cyclopentyl or cyclohexyl group,
- an aryl group having from 6 to 12 carbon atoms, preferably a phenyl or naphthyl group,
- an arylalkyl group having from 7 to 12 carbon atoms, preferably a benzyl group.

**15.** Process according to Claim 1, **characterized in that** the carboxylic acid of formula (III) is chosen from:

- acetic acid,
- acetic anhydride,
- propanoic acid,
- butanoic acid,
- pentanoic acid.

**16.** Process according to Claim 1, **characterized in that** the carboxylic acid of formula (II) is trifluoroacetic acid and the acid of formula (III) is acetic acid.

**17.** Process according to Claim 1, **characterized in that** the amount of the carboxylic acids employed is such that the ratio of the number of moles of carboxylic acid (III) to the number of moles of carboxylic acid (II) varies between 1 and 20, preferably between 3 and 8.

**18.** Process according to Claim 1, **characterized in that** the WHSV (weight hourly space velocity) is between 0.05 and 1.5 $h^{-1}$. preferably between 0.1 and 0.8 $h^{-1}$.

**19.** Process according to Claim 1, **characterized in that** use is made of a carrier gas or a mixture of gases which are

unreactive under the reaction conditions, preferably nitrogen, air, argon or helium.

**20.** Process according to Claim 19, **characterized in that** the ratio by volume of the carrier gas to the carboxylic acid (II) varies between 0 and 10, preferably between 0.1 and 2.0.

**21.** Process according to Claim 1, **characterized in that** use is made of an organic solvent, preferably an aprotic organic solvent, which dissolves the carboxylic acids.

**22.** Process according to Claim 1, **characterized in that** the temperature of the ketonization reaction is between 250°C and 450°C and preferably between 280°C and 300°C.

**23.** Process according to Claim 1, **characterized in that** the reaction pressure is atmospheric pressure.

**24.** Process according to Claim 1, **characterized in that** the starting reactants, namely the carboxylic acids, are vaporized and then, preferably entrained by a carrier gas, they are introduced over the catalyst.